# EUROPEAN PATENT APPLICATION

(11) **EP 1 378 559 A1**
(43) Date of publication of application: **07.01.2004**
(21) Application number: 02077667.0
(22) Date of filing: 05.07.2002
(51) Int. Cl.: C10G 70/06, C07C 7/00

(54) **Process for the recovery of ethylene and propylene**

(71) Applicant: DSM Hydrocarbons BV, 6135 KR Sittard (NL)
(72) Inventor: Hubbers, Theodorus Petrus Everardus Antonius, 6417 BR Heerlen (NL)
(74) Representative: Krijgsman, Willem

(57) **Abstract**

Process for the recovery of ethylene and propylene from a stream A originating from cracking a hydrocarbon feed stream. From that stream A, being substantially free of hydrogen, acetylenes and dienes, ethylene and propylene are chemically absorbed in a solvent containing a compound from a metal of group 10 or 11 of the Periodic Table of the Elements, followed by recovery of ethylene and propylene from said solvent by heating and/or by reducing the pressure.

## Description

The invention relates to a process for the recovery of ethylene and propylene from a stream A originating from cracking a hydrocarbon feed stream. Cracking a hydrocarbon feed stream to obtain ethylene and propylene is a well known process.

Hydrocarbon cracking can be performed thermally, in a steam cracker, and catalytically, in a catalytic cracker.
Some state of the art hydrocarbon cracking processes are described in Uhlmann's Encyclopedia of Industrial Chemistry, Vol. A 10, 1996, p. 45-93.
The separation system of the most commonly used process starts with a front-end demethanisation to free the stream originating from cracking a hydrocarbon feed stream from hydrogen and methane, whereafter the stream is separated in two separate streams; one being primarily a stream containing hydrocarbons with 2 carbon atoms (C2 fraction) and the other being a stream containing hydrocarbons with 2 or more carbon atoms (C3+). The C2 fraction is hydrogenated to remove acetylene and then fractionated to separate ethylene from ethane. The C3+ stream is further fractionated to remove heavy ends, such as C4 material or light distillate. The recovered hydrocarbon stream with 3 carbon atoms (C3 fraction) is hydrogenated to remove methylacetylene and propadiene and then separated in propylene and propane.
A disadvantage of the process according to the state of the art is that both the C2 and the C3+ stream have to be hydrogenated and further fractionated separately, so that investment in at least 2 hydrogenation units and a lot of other fractionation equipment is necessary.

It is now discovered that it is possible to recover ethylene and propylene directly from a combined hydrogenated C2 and C3 hydrocarbon stream.

This is achieved by chemically absorbing ethylene and propylene from stream A, being substantially free of hydrogen, acetylenes and dienes, in a solvent containing a compound from a metal of group 10 or 11 of the Periodic Table of the Elements, followed by recovery of ethylene and propylene from said solvent by heating and/or by reducing the pressure.

The advantage of the process according to the invention is that only one hydrogenation unit is necessary for treating the combined C2 and C3 hydrocarbon streams.

A further advantage of the process according to the invention is that the installation of expensive equipment for the fractionation of the C2 and C3 streams is not necessary anymore.

The Periodic Table of the Elements is here and hereafter the Periodic Table of the Elements (new IUPAC-notation) that can be found on the inside of the cover of the Handbook of Chemistry and Physics, 70^{th} edition, 1989/1990.

The process according to the present invention is directed to the recovery of ethylene and propylene from a stream A originating from cracking a hydrocarbon feed stream. Stream A has to be substantially free of hydrogen, acetylenes and dienes. Stream A can for instance be obtained by subjecting a hydrocarbon stream originating from cracking a hydrocarbon feed stream to demethanization, in order to remove methane and hydrogen from that stream, and to (partial) hydrogenation to free the hydrocarbon stream from acetylenes and dienes. Stream A primarily consists of ethylene, ethane, propylene and propane.
By subjecting stream A to a chemical absorption process ethylene and propylene are separated from ethane and propane.

The chemical absorption process is performed by bringing stream A in contact with a solvent containing a compound from a metal of group 10 or 11 of the Periodic Table of the Elements. Preferably, the metal compound comprises Cu or Ag. Examples of Ag-containing compounds are silver acetate, silver nitrate and silver tetrafluoroborate. Examples of Cu-containing compounds are cuprous nitrate, cuprous sulphate, cuprous tetrafluoroborate, cuprous tetrachloroaluminate and cuprous diketonate. Combinations of two or more compounds can be used.

The solvent used in the chemical absorption process is chosen from water or aromatic and olefinic solvents. Examples of aromatic and olefinic solvents are alfa-methylstyrene, benzene and toluene. Ag-containing compounds are normally used in combination with water as the solvent. Cu-containing compounds can be used with water as the solvent, but than also a stabilizer must be present, such as, for instance ammonia, pyridine or an alkanolamine. Cu-containing compounds are usually dissolved in aromatic or olefenic solvents.

Most preferably the solvent is water and the metal compound is AgNO₃.

The concentration of the compound in the solvent is between 2 and 15 mol/l. Stream A is contacted with the solvent for chemical absorption at a temperature between 0 and 50 °C and a pressure of 0.5 to 3.0 MPa.
Ethane and propane can be recycled to the hydrocarbon cracker.

Ethylene and propylene which are absorbed are recovered from the solvent by heating and/or by reducing the pressure. It is also possible to heat the solvent first, whereafter the pressure is reduced or vice versa.
Preferably, the temperature at which ethylene and propylene are released from the solvent is between 80 and 150 °C and the pressure is between 0,1 and 1,0 MPa. Thereafter this product stream is separated in an ethylene stream and a propylene stream by distillation at a pressure between 0,1 and 0,3 MPa and a temperature between - 60 and 100°C.

With the process according to the invention it is possible to obtain an ethylene product stream with a purity above 99,95% and a propylene product stream with a purity above 99,5%.
The invention is also directed to a unit for the recovery of ethylene and propylene from a stream A, to a hydrocarbon cracker, preferably a steam cracker, and to a method to modify an existing hydrocarbon cracker by providing it with a unit according to the invention.

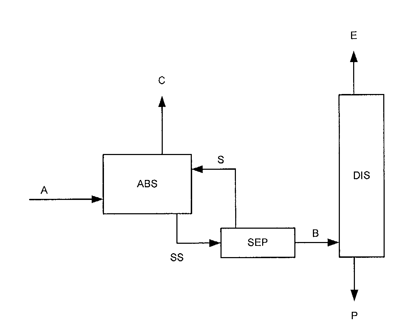

The invention will be further explained with respect to figure 1. Figure 1 describes an absorption unit according to the invention. To a chemical absorption unit (ABS) a stream A (A) consisting of 60 wt% ethylene, 30 wt% propylene, 1 wt% propane, 7 wt% ethane and 2 wt% methane was fed with an amount of 170 t/h and was contacted in this unit A with a solvent (S) containing AgNO3 at a temperature of 25 °C and a pressure of 0,1 MPa. The concentration of the AgNO3 in the solvent was 6 mol/l and the solvent was water. Ethylene and propylene were absorbed in the solvent and ethane and propane (C) were removed from ABS. C was recycled to a hydrocarbon cracker. The solvent containing ethylene and propylene (SS) was led to a separation unit wherein the pressure was released to 0,6 MPa. Thereafter the pressure in the separation unit was raised to 100 °C to remove ethylene and propylene from it whereafter S was recycled to ABS. The combined ethylene and propylene stream (B) was led to a distillation unit to separate B in ethylene (E) and propylene (P). The pressure during distillation was 1,8 MPa. The top temperature was -32 °C and the bottom temperature was 44°C.

An amount of 100 t/h of E was obtained with a purity of 99,999 wt% and an amount of 50,4 t/hr of P was obtained with a purity of 99,98 wt%.

## Claims

1. Process for the recovery of ethylene and propylene from a stream A originating from cracking a hydrocarbon feed stream, **characterised in that** from stream A, being substantially free of hydrogen, acetylenes and dienes, ethylene and propylene are chemically absorbed in a solvent containing a compound from a metal of group 10 or 11 of the Periodic Table of the Elements, followed by recovery of ethylene and propylene from said solvent by heating and/or by reducing the pressure.

2. Process according to claim 1, **characterised in that** the metal compound comprises Cu or Ag.

3. Process according to claim 1 or 2, **characterised in that** that the solvent is water and the metal compound is AgNO₃.

4. Process according to any one of claims 1-3, **characterised in that** the temperature at which ethylene and propylene are released from the solvent is between 80 and 150°C.

5. Process according to any one of claims 1-3, **characterised in that** the stream resulting from the chemical absorption is separated in an ethylene stream and a propylene stream by distillation.

6. Unit for the recovery of ethylene and propylene from a stream A originating from cracking a hydrocarbon feed stream, **characterised in that** the unit is a chemical absorption unit wherein a stream A is contacted with a solvent containing a compound from a metal of group 10 or 11 of the Periodic Table of the Elements, to absorb ethylene and propylene from this stream.

7. Unit according to claim 6, **characterised in that** the unit also comprises a unit wherein the solvent containing ethylene and propylene is heated and/or reduced in pressure to release the ethylene and propylene from the solvent.

8. Unit according to any one of claims 6-7, **characterised in that** the metal compound contains AgNO₃.

9. Hydrocarbon cracker comprising a unit according to any one of claims 6-8.

10. Hydrocarbon cracker according to claim 9, **characterised in that** the cracker is a steam cracker.

11. Hydrocarbon cracker according to any one of claims 9-10, **characterised in that** the feed to the cracker is naphta or gas oil.

12. Method to modify an existing hydrocarbon cracker by providing it with a unit according to any one of claims 6-8.
